(19) Europäisches Patentamt
European Patent Office
Office européen des brevets

(11) EP 4 729 606 A1

(12) **EUROPEAN PATENT APPLICATION**

(43) Date of publication:
**22.04.2026 Bulletin 2026/17**

(21) Application number: **24207280.9**

(22) Date of filing: **17.10.2024**

(51) International Patent Classification (IPC):
***C12N 5/00*** $^{(2006.01)}$ ***C12N 5/0783*** $^{(2010.01)}$

(52) Cooperative Patent Classification (CPC):
**A01N 1/125; C12N 5/0603; C12N 5/0618; C12N 5/0636; C12N 5/0693**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Sulfateq B.V.**
**9726 GN Groningen (NL)**

(72) Inventors:
- **VAN DER GRAAF, Adrianus Cornelis**
**9726 GN Groningen (NL)**
- **HENNING, Robert Henk**
**9726 GN Groningen (NL)**
- **SWART, Daniël Henri**
**9726 GN Groningen (NL)**
- **KRENNING, Guido**
**9726 GN Groningen (NL)**
- **RADOVIC, Marko**
**9726 GN Groningen (NL)**

(74) Representative: **Hoyng Rokh Monegier B.V.**
**Rembrandt Tower, 30th Floor**
**Amstelplein 1**
**1096 HA Amsterdam (NL)**

(54) **METHODS FOR THE CRYOPRESERVATION OF CELLS**

(57) The invention relates to certain chromanol, quinone or hydroquinone compounds and derivatives thereof for the protection of cells against cell injury caused by cryopreservation. In particular, the present invention relates to the protection of CAR-T cells during cryopreservation. Specifically, the present invention relates to chromanol compounds chosen from (6-hydroxy-2,5,7,8-tetramethylchroman-2-yl)(4-(2-hydroxyethyl)piperazin-1-yl)methanone, S-(6-hydroxy-2,5,7,8-tetramethylchroman-2-yl)(4-(2-hydroxyethyl)piperazin-1-yl)methanone or more the hydrochloric acid salt of S-(6-hydroxy-2,5,7,8-tetramethylchroman-2-yl)(4-(2-hydroxyethyl)piperazin-1-yl)methanone.

**FIG 1**

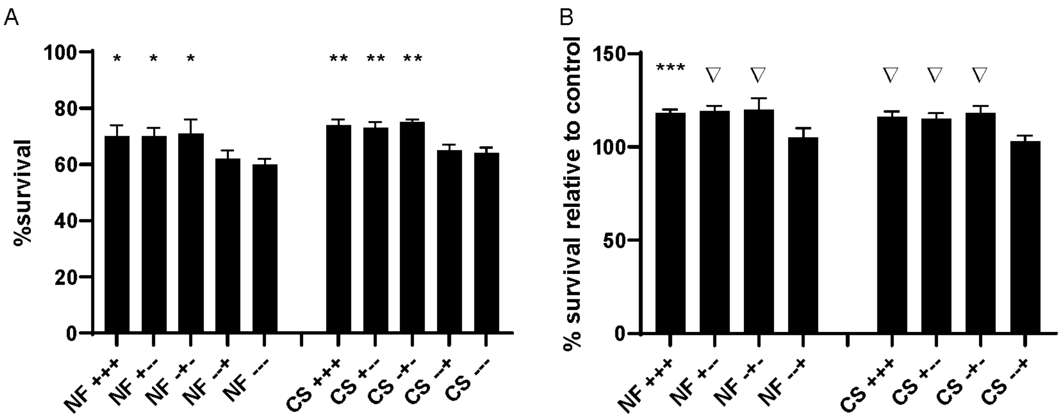


EP 4 729 606 A1

## Description

I. Field of the Invention

The invention relates methods for the protection of cells, in particular mammalian T-cells, against cell injury caused by cryopreservation, which comprises storing, freezing and thawing. Furthermore, the invention relates to the use of a compound according to formula (I) or (II), or a pharmaceutically acceptable salt thereof for the protection of cells, in particular mammalian T-cells, against cell injury caused by cryopreservation.

II. Description of the Background Art

Methods for the preservation of biological materials are employed in many clinical and veterinary applications wherein living material, including organs, tissues and cells, is harvested and stored in vitro for some period of time before use. Preservation techniques are also important in the storage of primary cells and cell lines for experimental use in hospital, industrial, university and other research laboratories.

One of the methods currently employed for the preservation of cellular biological materials is cryopreservation. Cryopreservation and its associated freezing and thawing procedures-short "freeze-thawing"-are among the final steps in economically viable manufacturing and clinical application of diverse cellular therapeutics. The goal is to store living biological materials for an extended period of time with minimal loss of normal biological structure and function.

The efficiency of the cryopreservation process is still partially compromised due to several factors. Reduced cell viability, increased senescence and impaired cellular functions are among the most widely reported adversities associated with oxidative stress generated during cryopreservation of cells. During cryopreservation cells experience damage during the freezing and thawing process. During the cooling phase of cryogenic preservation formation of intra- and extracellular ice crystals can damage the cell membrane and other structures. Moreover, dehydration of cells can occur. Furthermore, rewarming induces high cellular stress that results in cell death. Among such damage, the generation of supra-physiological levels of reactive oxygen species (ROS) can impair cellular functions and survival. Antioxidants are potential additives that have been reported to partially reverse freeze-thaw stress-associated impairments.

Hence, for the majority of mammalian cells, as is the case for the products and medications for cell therapy, whether the cells are genetically modified or not, it is vital to use cryoprotectants in order to preserve cell integrity and functionality. Dimethyl sulfoxide (DMSO, $Me_2SO$) has been the cryoprotectant of choice in most biobanking situations due to its exceptional performance in mitigating freezing-related damages. However, there is concern over the toxicity of DMSO and its potential side effects after administration to patients. At the same time, using DMSO will not prevent all damage.

There thus remains a need in the art for improved methods for the cryopreservation of living biological materials.

WO 2008/070235 relates to extender compositions comprising an oligomeric proanthocyanidin and an antioxidant bioflavonoid complex for the preservation of sperm cells and other reproductive media for use in artificial insemination.

EP2367419A1 relates to a composition for cryopreservation comprising Trolox, Na+, K+, Ca2+, Mg2+' Cl-, H2PO4 -, HEPES, lactobionate, sucrose, mannitol, glucose, dextran-40, adenosine, and glutathione.

It is an object of this invention to provide methods that can be used to protect cells, such as mammalian cells, in particular mammalian T-cells, against cell injury caused by cryopreservation.

III. Brief Summary of the invention

The above objects are met by providing certain chromanol, quinone or hydroquinone compounds. For the present invention, the compound according to formula (II) includes the hydrogenated quinone (i.e. the hydroquinone) analogue, although the quinone derivative is preferred in view of stability.

The above objects are further met by the present invention by providing a method for protection of cells against cell injury caused by cryopreservation comprising adding a compound to the cells, wherein the cells are selected from the group comprising lymphocytes, hematopoietic stem cells, mesenchymal stem cells, embryonic stem cells, and neural stem cells; and,

wherein the compound has the structural formula of (I) or (II), or a pharmaceutically acceptable salt thereof,

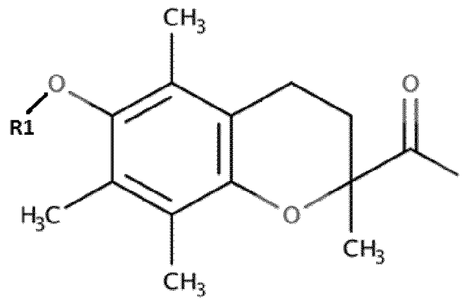

(I)

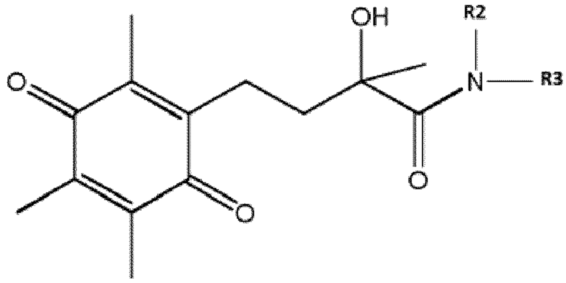

(II)

- wherein R1 represents a hydrogen or prodrug moiety that can be removed in living tissue
- and wherein either

  - ◦ R2 and R3 together with the N atom to which they are attached form a saturated or unsaturated, non-aromatic, optionally substituted 5-8 membered ring, having one to four N, O, or S atoms, wherein R2 and R3 together contain 3-12 carbon atoms;
  - ◦ or R2 is a hydrogen atom, or an alkyl group with 1-6 carbon atoms, and R3 is an alkyl group, optionally substituted with nitrogen or oxygen, wherein the alkyl group comprises 3-12 carbon atoms, the alkyl group in R3 comprises one or more non-aromatic cyclic structures and may contain linear and/or branched groups, and one or more ethylenic unsaturations.

In a preferred embodiment, R1 is hydrogen or forms together with the 6-oxygen an ester group with 2 - 6 carbon atoms.

In a preferred embodiment, in either compounds according to formula (I) or according to formula (II), R2 and R3 together with the N atom to which they are attached form a saturated ring incorporating an additional N atom, which ring is unsubstituted or substituted with an alcohol, or alkanol group having 1-4 carbon atoms.

In a preferred embodiment, the compound is a compound according to formula I. Preferably, R2 and R3 together with the N atom to which they are attached form a 5-7 membered ring comprising one additional amine group, which ring is optionally substituted with methyl, ethyl, or alcohol substituted methyl or ethyl.

In another preferred embodiment, R2 is a hydrogen atom and R3 comprises a saturated cyclic structure having 4-7 carbon atoms and having one nitrogen atom, which ring may be substituted with an alkyl group, alcohol group, or with a group with 1-4 carbon atoms that may comprise an oxygen, carboxylic acid or amine group. Preferably, the compound is a compound according to formula II and wherein R2 is a hydrogen atom and R3 comprises a cyclic structure having 4-6 carbon atoms and having one nitrogen atom which ring is optionally substituted with methyl, ethyl, or alcohol substituted methyl or ethyl.

In a preferred embodiment, the compound according to formula (I) or formula (II) has a molecular weight lower than 500 Da.

According to yet another preferred embodiment, the compound is (6-hydroxy-2,5,7,8-tetramethylchroman-2yl)(piperazin-1-yl)methanone (SUL-121), ((S)-6-hydroxy-2,5,7,8-tetramethyl-N-((R)-piperidin-3-yl)chroman-2-carboxamide hydrochloride (SUL-13) or (6-hydroxy-2, 5,7, 8-tetramethylchroman-2-yl)(4-(2-hydroxyethyl)piperazin-1-yl)methanone (SUL-109). Preferably, the compound is the S-enantiomer of SUL-109: S-(6-hydroxy-2,5,7,8-tetramethylchroman-2-yl)(4-(2-hydroxyethyl)piperazin-1-yl)methanone (SUL-138). More preferably, the compound is the hydrochloric acid salt of SUL-138 (SUL-238).

In a preferred embodiment according to the present invention, the protection of cells occurs during storage, which storage comprises a frozen state. Hence, at least part of the storage occurs at a temperature below the freezing point of the cell culture medium, in particular at about -20°C or lower, preferably at about -80°C or lower, and most preferably at about -196°C, the boiling point of liquid nitrogen.

In a preferred embodiment, the addition of the compound occurs prior to cooling the cells, which preferably is shortly before freezing. In yet another preferred embodiment, the addition of the compound occurs directly after thawing the cells, i.e. within 15 min or less after thawing, preferably within 5 min or less.

In a preferred embodiment, the cells are lymphocytes, preferably T cells, more preferably CAR-T cells.

Preferably, the addition of the compound occurs prior to freezing the cells, and additionally directly after thawing the cells.

Adding a compound to the cells is effected by adding the compound to the medium in which the cells are present and/or to the freeze dried cells. The compound will swiftly diffuse into the cells.

In one embodiment, compound is added in an amount of less than 100 $\mu$M, preferably less than 50 $\mu$M, more preferably between 0.1 $\mu$M and 10 $\mu$M of the medium with cells.

Additionally, the above objects are also met by the present invention by using certain chromanol, quinone or hydroquinone compounds according to formula (I) or according to formula (II) in protection against cryopreservation, wherein the cells are selected from the group comprising lymphocytes, hematopoietic stem cells, mesenchymal stem cells, embryonic stem cells, and neural stem cells.

## IV. Short description of the Figures

**FIG 1:** SUL-238 supplementation prior to or during freezing survival improves post. A) SUL-238 improves Jurkat absolute survivability 3-hour post thaw when added prior or during freezing. B) SUL-238 improves relative Jurkat 3-hour survival post thaw compared to NutriFreez® D10 Cryopreservation Medium (Sartorius) and CryoStor® CS10 Cell Freezing Medium (Sigma/Merck).

No significant difference in absolute and relative cell survival between NutriFreez® D10 Cryopreservation Medium (Sartorius) and CryoStor® CS10 Cell Freezing Medium (Sigma/Merck) was found.

**[0027]** * $p < 0.0009$; ** $p < 0.0001$; *** $p < 0.0044$; ∇ $p < 0.0001$ versus NF/CS --- (n=5), One-way ANOVA followed by Dunnett's multiple comparison test.

**FIG 2:** SUL-238 mediated improvements persist up to 24 hours post thaw. A) SUL-238 mediated post thaw survival improvements persist for up to 24 hours. B) Relative Jurkat survival 24-hour post thaw relative to non-supplemented NutriFreez® D10 Cryopreservation Medium (Sartorius) control * $p < 0.0341$ versus NF--- (n=2) One-way ANOVA followed by Dunnett's multiple comparison test.

**FIG 3:** SUL-238 improves post thaw recovery of Jurkat cells. Supplementation improves post thaw recovery of Jurkat cells in both NutriFreez® D10 Cryopreservation Medium (Sartorius) and CryoStor® CS10 Cell Freezing Medium (Sigma/Merck) 3 hours post thaw. For all conditions 2 million/ mL cells were frozen.

**[0030]** * $p < 0.0001$ versus NF/CS ---, (n=5) One-way ANOVA followed by Dunnett's multiple comparison test.

**FIG 4:** Growth curve for Jurkat cells post thaw. Cell count for the first 3 hours post thaw was elevated in both NutriFreez® D10 Cryopreservation Medium (Sartorius) and CryoStor® CS10 Cell Freezing Medium (Sigma/Merck) supplemented with SUL-238. For all conditions 2 million/ mL cells were frozen. Each SUL-238 condition is represented against the respective negative control.

N=5 for results up to 3 hours post thaw. N=2 for results up to 24 hours post thaw. N=1 for results beyond 48 hours.

## V. Detailed description of the invention

The above objects are met by the present invention by providing a method for protection of cells comprising adding a compound according to formula (I) or (II), as shown above, or a pharmaceutically acceptable salt thereof, wherein the cells are selected from the group comprising lymphocytes, hematopoietic stem cells, mesenchymal stem cells, embryonic stem cells, and neural stem cells.

### Compounds

In a preferred embodiment, R1 in formula (I) is hydrogen or forms together with the 6-oxygen an ester group with 2-6 carbon atoms. The ester can comprise one or more ether or alcohol groups. Suitable esters are acetate, butyrate, 3-hydroxy butyrate and the like.

In a preferred embodiment in either compounds according to formula (I) or according to formula (II), R2 and R3 together with the N atom to which they are attached form a saturated ring having 3-6 carbon atoms and incorporating one additional N atom, which may be substituted with 1-4 carbon atoms that may comprise an oxygen, carboxylic acid or amine group.

More preferably, R2 and R3 together with the N atom to which they are attached form a 5-7 membered ring comprising one additional amine group, which ring is optionally substituted with methyl, ethyl, or alcohol substituted methyl or ethyl.

In another preferred embodiment, R2 is a hydrogen atom and R3 comprises a cyclic structure having 3-6 carbon atoms and having one nitrogen atom.

More preferably, R2 is a hydrogen atom, and R3 comprises a 5-7 membered ring comprising one additional amine group, which ring is attached to the amide-nitrogen, and which ring is optionally substituted with methyl, ethyl, or alcohol substituted methyl or ethyl.

In either case, the ring (the cyclic structure formed by R2 and R3, or of R3 alone) may be unsubstituted or substituted with an alkyl having 1-4 carbon atoms, alcohol, or alkanol group having 1-4 carbon atoms, such as ethylol.

In a preferred embodiment according to the invention, the compound either according to formula (I) or according to formula (II) has a molecular weight lower than 500 Da.

Certain chromanol compounds have been described in WO2014/098586. The compounds described in detail have abbreviations, referring to SUL-XXX (XXX being a 2 or 3 digit number). Many of these compounds are racemic mixtures, although some enantiomers have been tested as well. Suitable methods to prepare chromanol compounds according to the present invention are described in WO2014/098586 or WO2014/011047.

WO 2017/060432 A1 discloses amide-derivatives of 2-hydroxy-2-methyl-4-(3,5,6-trimethyl-1,4-benzoquinon-2-yl)-butanoic acid and methods of making such compounds.

Hydrogenated quinone derivatives can be easily prepared by hydrogenation of the quinone structure.

According to yet another preferred embodiment, the compound is (6-hydroxy-2,5,7,8-tetramethylchroman-2yl)(piperazin-1-yl)methanone (SUL-121), ((S)-6-hydroxy-2,5,7,8-tetramethyl-N-((R)-piperidin-3-yl)chroman-2-carboxamide hydrochloride (SUL-13) or (6-hydroxy-2, 5,7, 8-tetramethylchroman-2-yl)(4-(2-hydroxyethyl)piperazin-1-yl)methanone (SUL-109). Preferably, the compound is the S-enantiomer of SUL-109: S-(6-hydroxy-2,5,7,8-tetramethylchroman-2-yl)(4-(2-hydroxyethyl)piperazin-1-yl)methanone (SUL-138).

The compounds are effective as a racemic mixture or in a substantially pure enantiomeric form. The compounds have one or more chiral centers, generally one or two.

Preferably, the compound is a substantially enantiomerically pure compound. Substantially enantiomerically pure is about 95% enantiomeric excess or more, more preferably about 98% enantiomeric excess, and most preferably about 99% or more enantiomeric excess. Also, in case the compound contains more than one chiral center, these amounts apply.

The counterion in the pharmaceutically acceptable salt can be a counterion as known in the art. Preferably, the compounds have at least one basic nitrogen, an amine, which can be protonated. The counterion preferably is a halogen such as chloride, sulphate, citrate, formate or the like, and most preferably chloride. More preferably, the compound is the hydrochloric acid salt of SUL-138 (SUL-238).

## Cells

The above objects are met by the present invention by providing a method for protection of cells comprising adding a compound to the cell medium comprising the cells.

The methods of the present invention may be used in the preservation of living biological materials including mammalian, plant and marine cells, cell lines, tissues and organs. When a living biological material is preserved, its viability is maintained in vitro for an extended period of time, such that the material resumes its normal biological activity on being removed from storage after cryopreservation.

Examples of mammalian biological materials which may be preserved using the present invention include, but are not limited to, cells and tissues such as bone marrow, embryos, lymphocytes (T-cells, B-cells and natural killer cells), osteoblasts, spermatozoa, granulocytes, dendritic cells, oocytes; and various animal cell lines established in tissue culture.

In one embodiment, the methods of the present invention are used in the protection of lymphocytes, hematopoietic stem cells, mesenchymal stem cells, embryonic stem cells, and neural stem cells.

Preferably, the methods according to the invention may be used in the protection of cells to be used in cell therapy. In particular, the methods of the present invention may be used for the protection of cells in cryopreservation, wherein the cells are selected from the group comprising lymphocytes, hematopoietic stem cells, mesenchymal stem cells, embryonic stem cells, and neural stem cells.

The methods according to the present invention are preferably used for the protection of lymphocytes. A lymphocyte is a type of white blood cell (leukocyte) in the immune system of most vertebrates. Lymphocytes include T cells, B cells and innate lymphoid cells, of which natural killer cells are an important subtype. The lymphocytes may also be engineered lymphocytes, preferably engineered T cells. Cellular T cell redirection by genetic modification of T cells with chimeric antigen receptors (CAR) or transgenic T cell receptors (TCR) is one of the important strategies employed in redirected T cell therapies, representing a new paradigm for cancer treatment. Hence, the methods according to the invention may be used for the protection of TCR-T cells and CAR-T cells, preferably CAR-T cells.

In one embodiment, the methods according to the invention may be used for the protection of natural or

engineered stem cells, or cells derived thereof. Preferably, the stem cells are selected from the group comprising hematopoietic stem cells, mesenchymal stem cells, embryonic stem cells, and neural stem cells. Mesenchymal stem cells, also known as mesenchymal stromal cells or medicinal signaling cells, are multipotent stromal cells that can differentiate into a variety of cell types, including osteoblasts, chondrocytes, myocytes and adipocytes. The mesenchymal stem cells may be derived from bone marrow, cord cells, adipose tissue, molar cells or amniotic fluid. Hematopoietic stem cells are the stem cells that give rise to other blood cells. Embryonic stem cells are pluripotent stem cells derived from the inner cell mass of a blastocyst, an early-stage pre-implantation embryo. Neural stem cells are somatic stem cells that give rise to neurons, astrocytes, and oligodendrocytes in the central nervous system.

The compounds described in the present invention have been found to be useful for protecting cells against injury caused by cryopreservation. Without being bound to theory, these compounds may also be used in the cryopreservation of biological materials that sustain injury from cryogenic storage, including tissues, organoids, embryos, gene therapy constructs, and the like.

## Cryopreservation medium composition

The inventors have found that cell survival increases with the addition of the compounds according to the invention to the preservative compositions used for the cryopreservation of cells.

In one embodiment the compound is added to the cells in an amount of less than 100 μM, preferably less than 50 μM, more preferably between 0.1 μM and 10 μM.

Conventional cryoprotectants preferably are also present in the preservative compositions. These cryoprotectants help protect cells from damage during the freezing and thawing process by preventing ice crystal formation, reducing osmotic stress, and stabilizing cell membranes and proteins. As used herein, the term "conventional cryoprotectants" refers to compounds including Dimethyl sulfoxide (DMSO), Glycerol, Ethylene glycol, Propylene glycol, Polyvinylpyrrolidone (PVP), Trehalose, Sucrose, Ficoll, Polyethylene glycol (PEG), Methanol, Proline, Serum albumin, Glutamine, Acetamide, Mannitol, Sorbitol, Dextrans, Methyl cellulose, Hydroxyethyl starch (HES), Cellulose derivatives, and the like. The absence of conventional cryoprotectants at concentrations greater than 10wt% is preferred, due to their toxic side effects. Preferably cryoprotectants are present in an amount of less than 10wt%, preferably less than 8wt%, more preferably less than 6wt%, even more preferably less than 4wt%. Preferably, conventional cryoprotectants are absent from the composition.

Buffering agents may also be present in the preservative composition. These buffering agents help maintain the pH stability of cell media during the cryopreservation process, ensuring an optimal environment for cell viability and function. The buffering agents suitable for use in the present invention include Phosphate-buffered saline (PBS), HEPES (4-(2-hydroxyethyl)-1-piperazineethanesulfonic acid), MOPS (3-(N-morpholino)propanesulfonic acid), Tris (tris(hydroxymethyl)aminomethane), Sodium bicarbonate (NaHCOs), Good's buffers (MES (2-(N-morpholino)ethanesulfonic acid), TES (N-tris(hydroxymethyl)methyl-2-aminoethanesulfonic acid), PIPES (piperazine-N,N'-bis(2-ethanesulfonic acid)), HEPPS (N-(2-hydroxyethyl)piperazine-N'-(3-propanesulfonic acid)), BES (N,N-bis(2-hydroxyethyl)-2-aminoethanesulfonic acid)), Citrate buffer, Acetate buffer, Glycine buffer, Succinate buffer, and the like.

Sugars may also be present in the preservative compositions. These sugars help stabilize cell membranes and proteins during the freezing and thawing process by replacing water molecules, reducing osmotic stress, and protecting cells from damage. The sugars suitable for use in the present invention include Sucrose, Trehalose, Glucose, Fructose, Mannose, Maltose, Galactose, Lactose, Raffinose, Sorbitol, Mannitol, Xylose, Arabinose, and the like.

Animal serum or serum replacements may also be present in the preservative compositions. These serums and serum replacements provide essential nutrients, growth factors, and proteins that support cell survival and proliferation during the cryopreservation process and subsequent thawing. The animal derived serums suitable for use in the present invention include Fetal Bovine Serum (FBS), Newborn Calf Serum (NCS), Human Serum, Horse Serum, Rabbit Serum, and the like. Serum replacements suitable for use in the present invention include KnockOut™ Serum Replacement, B27 Supplement, N2 Supplement, Nu-Serum™, BIT9500 (BSA, Insulin, Transferrin), CTS™ (Cell Therapy Systems) Serum Replacement, ULTROSER™ G, StemSpan™ Serum-Free Expansion Supplement, PlasmaLyte, Chemically Defined Lipid Concentrate, and the like.

Antioxidants may also be present in the preservative compositions. These antioxidants help protect cells from oxidative stress by neutralizing reactive oxygen species (ROS) generated during the freezing and thawing process, thereby improving cell viability and functionality. Antioxidants suitable for use in the present invention include Vitamin E (α-Tocopherol), Ascorbic Acid (Vitamin C), Glutathione, N-acetylcysteine (NAC), Superoxide Dismutase (SOD), Catalase, Butylated Hydroxytoluene (BHT), Butylated Hydroxyanisole (BHA), Trolox (a water-soluble vitamin E analog), Melatonin, Lipoic Acid, Coenzyme Q10 (CoQ10), Epigallocatechin Gallate (EGCG, found in green tea), Quercetin, Resveratrol, Curcumin, Flavonoids, Selenium, Beta-Carotene, and the like.

Amino acids may also be present in the preservative compositions. Amino acids play crucial roles in stabilizing cell structures, supporting cellular metabolism, and enhancing post-thaw recovery and function of cryopreserved cells.

Amino acids suitable for use in the present invention include L-Glutamine, Glycine, L-Alanine, L-Arginine, L-Asparagine, L-Aspartic Acid, L-Cysteine, L-Glutamic Acid, L-Glutathione, L-Histidine, L-Isoleucine, L-Leucine, L-Lysine, L-Methionine, L-Phenylalanine, L-Proline, L-Serine, L-Threonine, L-Tryptophan, L-Tyrosine, L-Valine and the like.

Antibiotics may also be present in the preservative compositions. Antibiotics help maintain a sterile environment by inhibiting the growth of bacteria and fungi in cell media compositions. Antibiotics suitable for use in the present invention include Penicillin, Streptomycin, Gentamicin, Amphotericin B, Kanamycin, Neomycin, Tetracycline, Ciprofloxacin, Vancomycin, Nystatin, G418 (Geneticin), Rifampicin, Chloramphenicol, Amoxicillin, Cefotaxime, Puromycin, Zeocin, Blasticidin, Hygromycin B, and the like.

Growth factors may also be present in the preservative compositions. Growth factors support various cellular processes, including proliferation, differentiation, and survival, which are essential for maintaining healthy and functional cell cultures. Growth factors suitable for use in the present invention include EGF, FGF, TGF-β, VEGF, PDGF, IGF, HGF, BMPs, NGF, BDNF, GDNF, Interleukins, G-CSF, GM-CSF, SCF, Erythropoietin, Thrombopoietin, LIF, EGFL, and the like.

Salts and ions may also be present in the preservative compositions according to the invention. Salts and ions are crucial for maintaining cellular homeostasis, regulating enzyme activity, and supporting cellular metabolism and other physiological processes. Preferably, calcium is present in the preservative compositions. Calcium is known to stabilize phospholipid bilayers found in cell membranes and to stabilize intercellular adhesion. Preferably the calcium is present as calcium sulfate or calcium chloride, and is present at a concentration greater than about 1.5 mM or less than about 2.0 mM, more preferably at a concentration of between about 1.5 mM and about 2.0 mM, and most preferably about 1.75 mM. The preservative compositions may include either sodium sulfate or sodium citrate.

## Storage conditions and method for storing cells

The inventors found that the compounds protect the cells against cell injury, which may eventually lead to cell death via necrosis or apoptosis. The compounds according to the invention provide protection against cell injury caused by cryopreservation. The protection can be provided by using the compound before, during, or after the frozen state. Cells cryopreserved using compounds according to the invention have a decreased cell death compared with cells stored without the compound.

Preferably, the present method is an *ex vivo* method. Accordingly, the compound can be used for protection during cryopreservation of cells. The storage can occur at a temperature which is suitable for storage of the particular cells and can be below the freezing point or lower, preferably below -80°C, even more preferably below -140°C. In a preferred embodiment, storage occurs in liquid nitrogen ($LN_2$), at about -196°C.

The inventors found that the above compounds protect the cells against cell injury during and after cooling down, and especially against injury that occurs during warming up back to the functional temperature. More cells survive these stress conditions and thus the storability increases when the cells are stored together with the compound according to the invention, compared with cells that are stored without the addition of a compound according to the invention.

The cells to be preserved are harvested using standard techniques and contacted, preferably immersed, in an aqueous preservative solution comprising a compound according to the present invention. Said compound may be added to the cells in an amount of less than 100 μM, preferably less than 50 μM, more preferably between 0.1 μM and 10 μM. The cells may be rinsed with the cryopreservation medium prior to immersion. While the cells may be stored at temperatures below freezing, including temperatures as low as about -196° C., materials may be conveniently stored at temperatures of about 4° C or lower.

Before the actual cryopreservation begins, cells are generally mixed with a cryopreservation medium. The compounds according to the invention preferably are added to the cells in this step and the compound can be part of the cryopreservation medium.

Cells mixed with cryopreservation medium are placed in cryovials or suitable containers. The samples are typically cooled to a temperature just above freezing (0°C to 4°C) before initiating the slow cooling process. This pre-cooling step helps to minimize thermal shock.

Thereafter, the sample comprising the cells is cooled at a rate of about 1°C per minute. During this step, the sample may be placed in a programmable freezer or a controlled-rate freezer.

Once the sample reaches a temperature of between -80°C to -150°C, it is transferred to a storage container, such as a cryovial or a cryo-tube, and then placed in liquid nitrogen. Liquid nitrogen maintains a temperature of about -196°C.

For further use of the biological material, the cryopreserved sample is rapidly thawed by immersing it in a 37°C water bath for a few minutes. After thawing, the preservative solution may be removed from the material and replaced with a suitable growth medium, or the stored material may be used directly in its preservative solution. The suitable growth medium preferably also comprises a compound according to the invention.

The technical effects and advantages of the various embodiments and aspects of the methods of the invention correspond *mutatis mutandis* to those described for the products of the invention and vice versa.

This then generally describes the invention but to assist with understanding, reference will now be made to the

accompanying comparison and non-limiting examples and figures which show embodiments of the invention.

## VI. Examples

This example shows that SUL 238 supplementation of established cryopreservation media, NutriFreez® D10 Cryopreservation Medium (Sartorius) and CryoStor® CS10 Cell Freezing Medium (Sigma/Merck), improves post thaw cell survival. Four different approaches for supplementation with SUL-238 were tested:

(1) Pre-treatment for 24 hours via supplementation of the cell culture medium.
(2) During freezing via supplementation of the cryopreservation medium.
(3) Post-thaw supplementation of culture media.
(4) Continued supplementation of the culture media prior to freezing (1) and after thawing (3), and supplementation of the cryopreservation media (2).

### Methods

E6-1 Jurkat T-cells, obtained from the University Medical Center Groningen, were cultured at 37 degrees Celsius 5% $CO_2$ using Gibco™ RPMI 1640 culture medium (Thermo Fisher Scientific), 10% Fetal Bovine Serum (Thermo Fisher Scientific) and 1% Penicillin/Streptomycin solution (VWR).

Jurkat cells were treated with1.0 μM SUL-238 (Sulfateq) at different stages of the freezing procedure. SUL-238 was prepared fresh every day in $dH_2O$ and filter sterilized. Control tests were done with no supplementation.

Jurkat cells were treated 24 hours prior to, during or after freezing by supplementing NutriFreez® D10 Cryopreservation Medium (Sartorius) or CryoStor® CS10 Cell Freezing Medium (Sigma/Merck) with SUL-238. After 24-hour treatment, cells were frozen at a concentration of 2 million/ml in cryovials (VWR) at controlled freezing rate of -1°C/min to -80°C using a Mr. Frosty™ Freezing Container (Thermo Fisher Scientific). Subsequently, the cryovials (VWR) were transferred to liquid nitrogen storage for five to seven days. Jurkat cells were thawed using a water bath at 37°C for 2-3 minutes, until all ice was visibly dissolved. Cell suspensions were immediately transferred to pre-warmed culture medium using a 1:5 dilution rate. The cell suspension dilutions were centrifuged at 400G for 5 min. Supernatant was removed from the cells and replaced with fresh warm culture medium. After thawing, the cells were counted using a Bürker-Türk hematocyte counting chamber. Live/Dead distinctions were made using Trypan Blue 0.4% (VWR) at a 1:1 dilution after incubating for 2-3 minutes. Absolute survival is expressed as the percentage of live cells relative to the total number of cells:

$$absolute\ survival = \frac{live\ cells}{live\ cells\ +\ dead\ cells} * 100\%$$

Relative survival is expressed as the percentage of survival cells in the sample relative to the survival cells in the untreated control:

$$relative\ survival = \frac{survival_{sample}}{survival_{control}} * 100\%$$

Statistical analysis was preformed via GraphPad Prism v8. One-way ANOVA followed by Dunnett's multiple comparisons. A p-value of $< 0.05$ was considered significant.

### Results

SUL-238 supplementation of NutriFreez® D10 Cryopreservation Medium (Sartorius) and CryoStor® CS10 Cell Freezing Medium (Sigma/Merck) improved post thaw survival and recovery of Jurkat cells (FIG 1). Absolute and relative survival improved at least up to 24 hours post thaw (**FIG 2**). Cell recovery post thaw was superior in SUL-238 supplemented cryopreservation media compared to non-supplemented cryopreservation media (**FIG 3**). Cell count for the first 3 hours post thaw was elevated for both NutriFreez and CryoStor supplemented with SUL-238 (**FIG 4**).

Figures are annotated as follows:

| Condition | Annotation in graph |
|---|---|
| Without SUL-238 | --- |
| SUL-238 present prior to freezing | --+ |
| SUL-238 present during freezing | -+- |
| SUL-238 present during thawing | +-- |
| SUL-238 present throughout | +++ |

## Claims

1. Method for protection of cells from damage caused by cryopreservation, comprising adding a compound to the cells,

   wherein the cells are selected from the group consisting of lymphocytes, hematopoietic stem cells, mesenchymal stem cells, embryonic stem cells, and neural stem cells; and,
   wherein the compound has the structural formula of (I) or (II), or a pharmaceutically acceptable salt thereof,

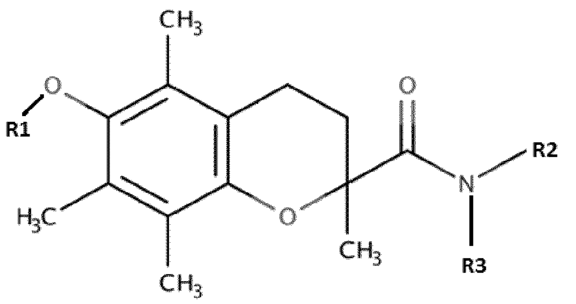

(I)

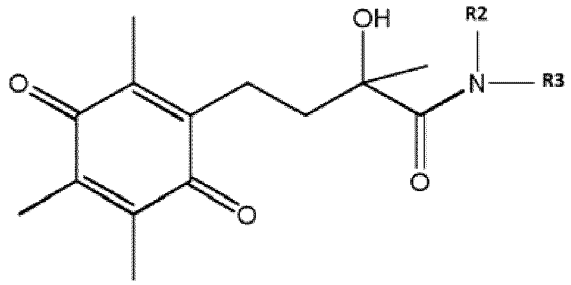

(II)

   - wherein R1 represents a hydrogen or prodrug moiety that can be removed in living tissue
   - and wherein either

     ◦ R2 and R3 together with the N atom to which they are attached form a saturated or unsaturated, non-aromatic, optionally substituted 5-8 membered ring, having one to four N, O, or S atoms, wherein R2 and R3 together contain 3-12 carbon atoms;
     ◦ or R2 is a hydrogen atom, or an alkyl group with 1-6 carbon atoms, and R3 is an alkyl group, optionally substituted with nitrogen or oxygen, wherein the alkyl group comprises 3-12 carbon atoms, the alkyl group in R3 comprises one or more non-aromatic cyclic structures and may contain linear and/or branched groups, and one or more ethylenic unsaturations.

2. Method according to claim 1, wherein R1 is hydrogen or forms together with the 6-oxygen an ester group with 2 - 6 carbon atoms.

3. Method according to any one of the preceding claims, wherein in either compounds according to formula (I) or according to formula (II), R2 and R3 together with the N atom to which they are attached form a saturated ring incorporating an additional N atom, which ring is unsubstituted or substituted with an alcohol, or alkanol group having

1-4 carbon atoms.

**4.** Method according to any one of the preceding claims, wherein the compound is a compound according to formula I.

**5.** Method according to claim 4, wherein R2 and R3 together with the N atom to which they are attached form a 5-7 membered ring comprising one additional amine group, which ring is optionally substituted with methyl, ethyl, or alcohol substituted methyl or ethyl.

**6.** Method according any one of claims 1-2, R2 is a hydrogen atom and R3 comprises a saturated cyclic structure having 4-7 carbon atoms and having one nitrogen atom, which ring may be substituted with an alkyl group, alcohol group, or with a group with 1-4 carbon atoms that may comprise an oxygen, carboxylic acid or amine group.

**7.** Method according to claim 6, wherein the compound is a compound according to formula II and wherein R2 is a hydrogen atom and R3 comprises a cyclic structure having 4-6 carbon atoms and having one nitrogen atom which ring is optionally substituted with methyl, ethyl, or alcohol substituted methyl or ethyl.

**8.** Method according to claim 1, wherein the compound is (6-hydroxy-2,5,7,8-tetramethylchroman-2yl)(piperazin-1-yl) methanone (SUL-121), ((S)-6-hydroxy-2,5,7,8-tetramethyl-N-((R)-piperidin-3-yl)chroman-2-carboxamide hydrochloride (SUL-13) or (6-hydroxy-2, 5,7, 8-tetramethylchroman-2-yl)(4-(2-hydroxyethyl)piperazin-1-yl)methanone (SUL-109).

**9.** Method according to claim 8, wherein the compound is the S-enantiomer of SUL-109: S-(6-hydroxy-2, 5,7, 8-tetramethylchroman-2-yl)(4-(2-hydroxyethyl)piperazin-1-yl)methanone (SUL-138), preferably wherein the compound is the hydrochloric acid salt of SUL-138 (SUL-238).

**10.** Method according to any one of claims 1-7, wherein the compound according to formula (I) or formula (II) has a molecular weight lower than 500 Da.

**11.** Method according to any one of the preceding claims, wherein the cells are lymphocytes, preferably T cells, more preferably a CAR-T cells.

**12.** Method according to any one of the preceding claims, wherein the cells are kept in a cell medium at a temperature below the freezing point of the cell medium or lower, preferably at about -20°C or lower, and more preferably at about -80°C or lower, and most preferably at about -196°C.

**13.** Method according to any one of the preceding claims, wherein the addition of the compound occurs prior to freezing the cell.

**14.** Method according to any one of claims 1-13, wherein the addition of the compound occurs after thawing the cell, preferably within 15 minutes after thawing the cells.

**15.** Method according to any one of the preceding claims, wherein the compound is added in an amount of less than 100 $\mu$M, preferably less than 50 $\mu$M, more preferably between 0.1 $\mu$M and 10 $\mu$M.

**16.** Use of a compound according to claim 1-10, for storing cells at a temperature below the freezing temperature of the cell medium, preferably at about -20°C or lower and more preferably of about -80°C or lower, most preferably at about -196°C, wherein the cells are selected from the group consisting of lymphocytes, hematopoietic stem cells, mesenchymal stem cells, embryonic stem cells, and neural stem cells.

**17.** Use of a compound according to any one of claims 16, wherein the cells are lymphocyte cells, preferably T-cells, more preferably CAR-T cells.

FIG 1

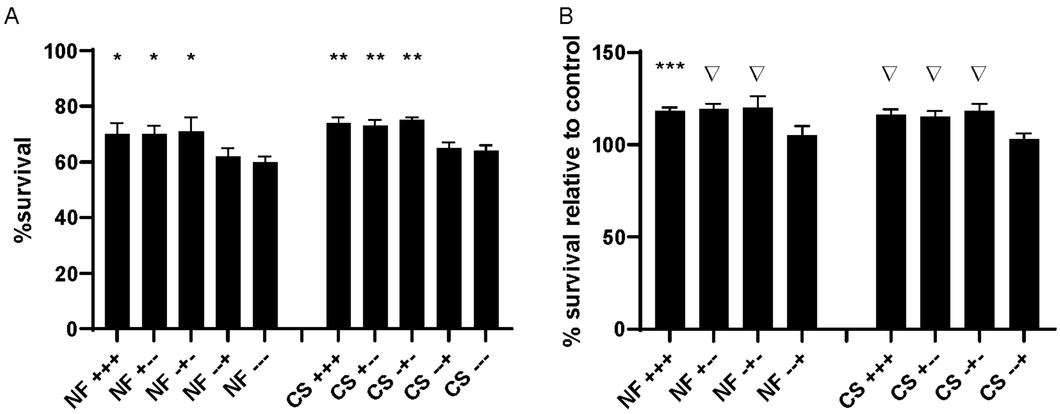
A
%survival
100
80
60
40
20
0
* * *
** ** **
NF +++
NF +--
NF -+-
NF --+
NF ---
CS +++
CS +--
CS -+-
CS --+
CS ---
B
% survival relative to control
150
100
50
0
***
▽ ▽ ▽ ▽ ▽
NF +++
NF +--
NF -+-
NF --+
CS +++
CS +--
CS -+-
CS --+

FIG 2

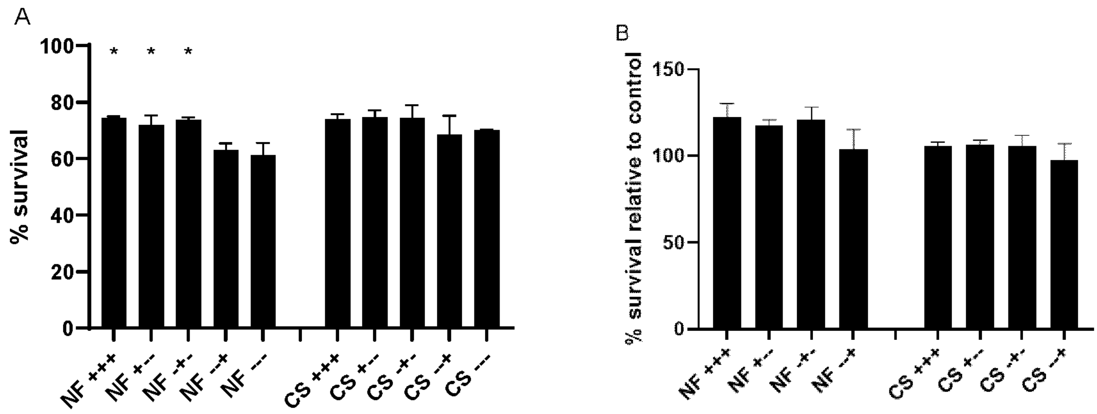
A
100
80
60
40
20
0
% survival
* * *
NF +++
NF +--
NF -+-
NF --+
NF ---
CS +++
CS +--
CS -+-
CS --+
CS ---
B
150
100
50
0
% survival relative to control
NF +++
NF +--
NF -+-
NF --+
CS +++
CS +--
CS -+-
CS --+

FIG 3

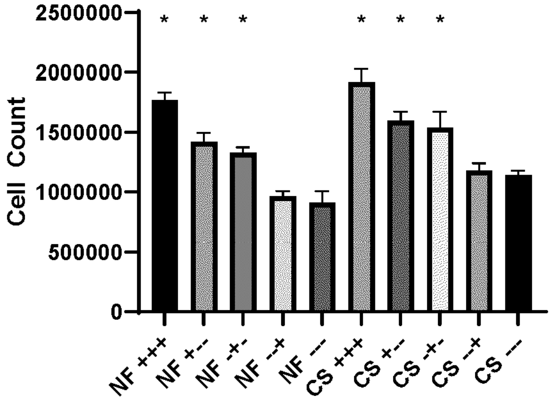
2500000
2000000
1500000
1000000
500000
0
Cell Count
*
*
*
*
*
*
NF +++
NF +--
NF -+-
NF --+
NF ---
CS +++
CS +--
CS -+-
CS --+
CS ---

**FIG 4**

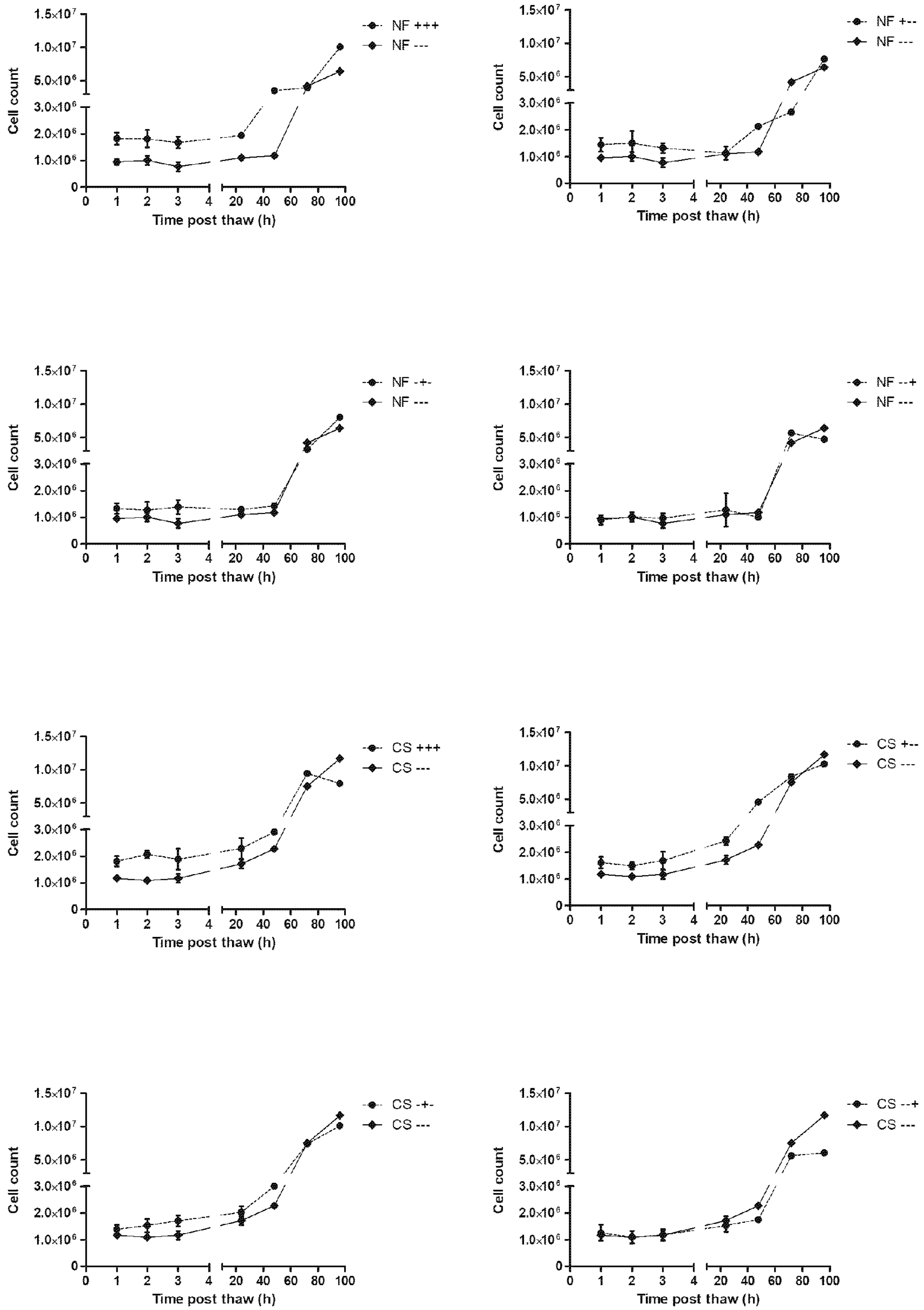
NF +++
NF ---
NF +--
NF ---
NF -+-
NF ---
NF --+
NF ---
CS +++
CS ---
CS +--
CS ---
CS -+-
CS ---
CS --+
CS ---
Cell count
1.5x10^7
1.0x10^7
5.0x10^6
3.0x10^6
2.0x10^6
1.0x10^6
0
0 1 2 3 4 20 40 60 80 100
Time post thaw (h)

Europäisches Patentamt
European Patent Office
Office européen des brevets

# EUROPEAN SEARCH REPORT

Application Number
EP 24 20 7280

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/131428 A1 (BEIJING ZEPHYRM BIOTECHNOLOGY CO LTD [CN]) 27 June 2024 (2024-06-27) * claim 1 * ----- | 1-10,12, 13,16 | INV. C12N5/00 C12N5/0783 |
| Y | AERTS-KAYA FATIMA S F ET AL: "SUL-109 Protects Hematopoietic Stem Cells from Apoptosis Induced by Short-Term Hypothermic Preservation and Maintains Their Engraftment Potential", BIOLOGY OF BLOOD AND MARROW TRANSPLANTATION, KLUGE CARDEN JENNINGS PUBLISHING, CHARLOTTESVILLE, VA, US, vol. 26, no. 4, 7 January 2020 (2020-01-07), pages 634-642, XP086151331, ISSN: 1083-8791, DOI: 10.1016/J.BBMT.2019.12.770 [retrieved on 2020-01-07] * Title, Abstract * ----- | 1-17 | |
| Y | WO 2014/098586 A1 (SULFATEQ BV [NL]) 26 June 2014 (2014-06-26) * claim 5 * * table 2 * ----- -/-- | 1-17 | TECHNICAL FIELDS SEARCHED (IPC) C12N A01N |

The present search report has been drawn up for all claims

1

| Place of search | Date of completion of the search | Examiner |
|---|---|---|
| Munich | 5 March 2025 | Antoni, Frauke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 20 7280

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TOLOUEE MARZIYEH ET AL: "Cooling of Cells and Organs Confers Extensive DNA Strand Breaks Through Oxidative Stress and ATP Depletion", CELL TRANSPLANTATION, vol. 31, 8 July 2022 (2022-07-08), XP093256040, US ISSN: 0963-6897, DOI: 10.1177/09636897221108705 Retrieved from the Internet: URL:https://journals.sagepub.com/doi/pdf/10.1177/09636897221108705> * Title, Abstract * ----- | 1-17 | |
| Y | JÜTTNER ANNIKA A. ET AL: "The modified 6-chromanol SUL-238 protects against accelerated vascular aging in vascular smooth muscle Ercc1 -deficient mice", THE JOURNAL OF CARDIOVASCULAR AGING, 13 October 2024 (2024-10-13), XP093256057, ISSN: 2768-5993, DOI: 10.20517/jca.2024.10 Retrieved from the Internet: URL:https://www.oaepublish.com/articles/jca.2024.10> * Abstract * ----- | 1-17 | TECHNICAL FIELDS SEARCHED (IPC) |
| Y | WO 2019/133992 A1 (EVERGREEN BIOSCIENCES [US]) 4 July 2019 (2019-07-04) * claims 34, 16 * * paragraph [0156] * ----- | 1-17 | |

The present search report has been drawn up for all claims

1

| Place of search | Date of completion of the search | Examiner |
|---|---|---|
| Munich | 5 March 2025 | Antoni, Frauke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 20 7280

This annex lists the patent family members relating to the patent documents cited in the above-mentioned European search report. The members are as contained in the European Patent Office EDP file on
The European Patent Office is in no way liable for these particulars which are merely given for the purpose of information.

05-03-2025

| Patent document cited in search report | Publication date | Patent family member(s) | Publication date |
|---|---|---|---|
| WO 2024131428 A1 | 27-06-2024 | CN 118217308 A | 21-06-2024 |
| | | WO 2024131428 A1 | 27-06-2024 |
| WO 2014098586 A1 | 26-06-2014 | AU 2013364538 A1 | 09-07-2015 |
| | | CA 2895701 A1 | 26-06-2014 |
| | | CY 1120682 T1 | 11-12-2019 |
| | | DK 2935232 T3 | 17-09-2018 |
| | | EP 2935232 A1 | 28-10-2015 |
| | | EP 3388426 A2 | 17-10-2018 |
| | | ES 2686599 T3 | 18-10-2018 |
| | | ES 2954292 T3 | 21-11-2023 |
| | | HR P20181434 T1 | 19-10-2018 |
| | | HU E039373 T2 | 28-12-2018 |
| | | JP 6359558 B2 | 18-07-2018 |
| | | JP 6684861 B2 | 22-04-2020 |
| | | JP 2016511743 A | 21-04-2016 |
| | | JP 2018162280 A | 18-10-2018 |
| | | LT 2935232 T | 25-09-2018 |
| | | MX 371337 B | 27-01-2020 |
| | | NL 2010010 C2 | 23-06-2014 |
| | | PL 2935232 T3 | 31-12-2018 |
| | | PL 3388426 T3 | 27-12-2023 |
| | | PT 2935232 T | 11-10-2018 |
| | | SI 2935232 T1 | 30-10-2018 |
| | | SM T201800463 T1 | 09-11-2018 |
| | | US 2015342174 A1 | 03-12-2015 |
| | | US 2019098890 A1 | 04-04-2019 |
| | | US 2021227822 A1 | 29-07-2021 |
| | | WO 2014098586 A1 | 26-06-2014 |
| | | ZA 201504602 B | 26-07-2017 |
| WO 2019133992 A1 | 04-07-2019 | AU 2018395543 A1 | 23-07-2020 |
| | | CA 3088844 A1 | 04-07-2019 |
| | | CN 111655258 A | 11-09-2020 |
| | | EP 3731841 A1 | 04-11-2020 |
| | | IL 275739 A | 31-08-2020 |
| | | JP 7594441 B2 | 04-12-2024 |
| | | JP 2021509121 A | 18-03-2021 |
| | | KR 20200118422 A | 15-10-2020 |
| | | SG 11202006304S A | 29-07-2020 |
| | | US 2019201381 A1 | 04-07-2019 |
| | | WO 2019133992 A1 | 04-07-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008070235 A
- EP 2367419 A1
- WO 2014098586 A
- WO 2014011047 A
- WO 2017060432 A1